**Europäisches Patentamt**

**European Patent Office**

**Office européen des brevets**

(19)

(11) Veröffentlichungsnummer: **0 473 131 A2**

# EUROPÄISCHE PATENTANMELDUNG

(12)

(21) Anmeldenummer: **91114443.4**

(22) Anmeldetag: **28.08.91**

(51) Int. Cl.⁵: **A61N 1/36**, A61N 1/05

(30) Priorität: **29.08.90 DE 4027266**

(43) Veröffentlichungstag der Anmeldung:
**04.03.92 Patentblatt 92/10**

(84) Benannte Vertragsstaaten:
**AT BE CH DE DK ES FR GB GR IT LI LU NL SE**

(71) Anmelder: **Wetzel, Horst**
**Holteistrasse, 10**
**W-3000 Hannover 1(DE)**

(72) Erfinder: **Barsom,Shafik Dr.Med.**
**Gabelstrasse 54**
**W-3000 Hannover(DE)**

(74) Vertreter: **König, Norbert, Dipl.-Phys. Dr.**
**Patentanwälte Leine & König**
**Burckhardtstrasse 1**
**W-3000 Hannover 1(DE)**

(54) **Elektrischer Stimulator für die Behandlung der weiblichen Inkontinenz.**

(57) Ein elektrischer Stimulator für die Behandlung der weiblichen Inkontinenz weist ein in die Vagina einführbares Gehäuse mit einer Schaltungsanordnung zur Erzeugung elektrischer Spannungsimpulse auf. Diese Spannungsimpulse werden über gehäuseaußenseitig angeordnete Elektroden abgegeben. Um den Stimulator betriebssicherer zu machen, befindet sich in der Verbindungsleitung zwischen einer im Gehäuse angeordneten Batterie und der Schaltungsanordnung ein in Ruhestellung geschlossener Reed-Kontakt. Das Gehäuse des Stimulators besteht aus zwei zusammenschraubbaren Gehäuseteilen, die an ihren offenen enden ringförmige Elektrodenteile aufweisen, die beim Zusammenschrauben der beiden Gehäuseteile miteinander in Kontakt gebracht werden und eine erste Elektrode bilden. Eine zweite Elektrode befindet sich auf dem die Schaltungsanordnung aufweisenden Gehäuseteil. Zum Ausschalten des Stimulators bei Nichtgebrauch dient eine Einrichtung, in die der Stimulator einlegbar ist und die einen Magneten zum öffnen des Reed-Kontaktes in einer ersten Position des Stimulators innerhalb dieser Einrichtung aufweist. In einer zweiten Position des Stimulators innerhalb der Einrichtung, in der der Reed-Kontakt außer Wirkverbindung mit dem Magneten steht, stehen zwei Kontakte mit den Elektroden des Stimulators in Verbindung. Die Kontakte sind zur Funktionskontrolle an eine Anzeigeeinrichtung für die durch die Schaltungsanordnung erzeugten Spannungsimpulse angeschlossen.

Die Erfindung betrifft einen elektrischen Stimulator für die Behandlung der weiblichen Inkontinenz gemäß Oberbegriff des Anspruchs 1.

Ein solcher Stimulator ist durch die DE-OS 38 27 232 bekannt. Dieser bekannte Stimulator weist einen im Gehäuse angeordneten, von außen zu bedienenden Schalter zum Ein- und Ausschalten des Stimulators auf. Da der Stimulator regelmäßig sterilisiert werden muß, besteht die Gefahr, daß ein solcher Schalter beschädigt und funktionsunfähig wird. Außerdem besitzt der bekannte Stimulator keine geeignete Funktionskontrolle. Die Massekontaktgabe erfolgt störanfällig über einen am Gehäuseende befindlichen Schraubdeckel. Insgesamt ist daher die Gefahr von Betriebsstörungen beim bekannten Stimulator relativ groß.

Die Aufgabe der vorliegenden Erfindung besteht darin, einen elektrischen Stimulator der eingangs genannten Art so zu verbessern, daß Betriebsstörungen durch Sterilisationsvorgänge nicht mehr auftreten können und eine Funktionskontrolle des Gerätes möglich ist.

Diese Aufgabe wird durch die Ausbildung gemäß Kennzeichen des Anspruchs 1 gelöst.

Die erfindungsgemäße Aufgabenlösung sieht den Einsatz von Reed-Kontakten innerhalb des Gehäuses vor, die in Ruhestellung geschlossen sind und bei Einwirkung eines Magnetfeldes geöffnet werden. Dies hat den Vorteil, daß die Ein-/Ausschalteinrichtung durch den Sterilisationsvorgang nicht beschädigt werden kann und damit außer Funktion gesetzt werden kann. Das Ausschalten und somit die Stromunterbrechung für die Schaltungsanordnung erfolgt durch einen Magneten, der sich in einer zur Aufbewahrung des Stimulators vorgesehenen Einrichtung befindet. Diese Einrichtung weist ferner eine Anzeigeeinrichtung für die von der Schaltungsanordnung erzeugten Spannungsimpulse auf, so daß hierüber eine Funktionskontrolle möglich ist.

Vorteilhafte und zweckmäßige Weiterbildungen der erfindungsgemäßen Aufgabenlösung sind in den Unteransprüchen gekennzeichnet.

Die Erfindung soll nachfolgend anhand der beigefügten Zeichnung, die ein Ausführungsbeispiel zeigt, näher erläutert werden.

Es zeigt:

Fig. 1 schematisch den Aufbau eines Stimulators und

Fig. 2 schematisch eine Schatulle zur Aufnahme, zum Ausschalten und zur Funktionskontrolle des Stimulators bei Nichtgebrauch.

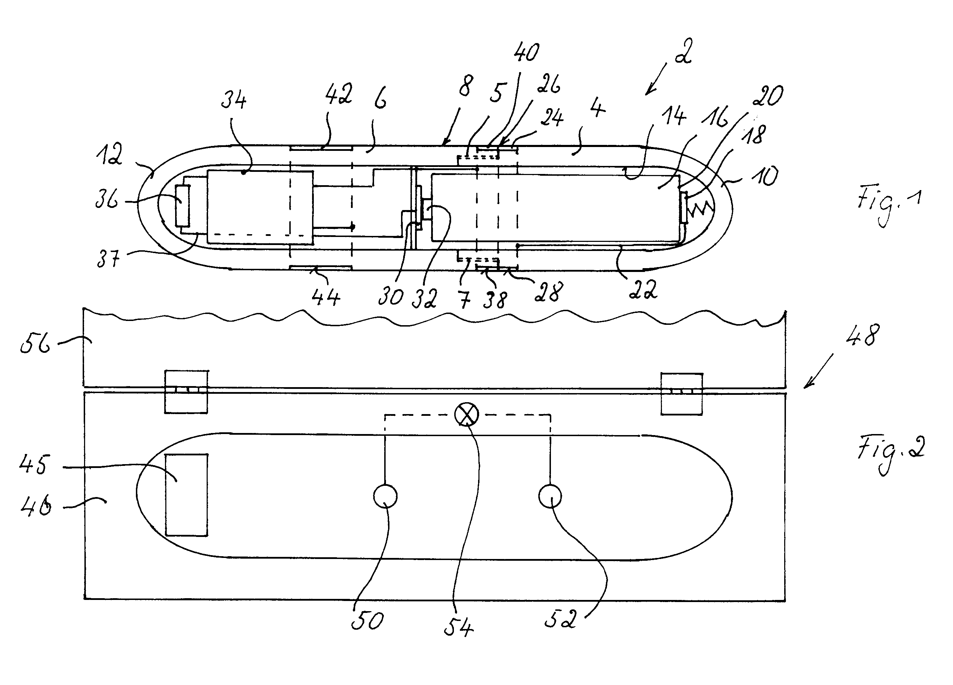

Die Zeichnung zeigt einen elektrischen Stimulator 2 mit einem aus zwei zusammenschraubbaren Gehäuseteilen 4, 6 bestehenden zylindrischen, geschlossenen Gehäuse 8 mit abgerundeten Enden 10, 12, wobei das eine Gehäuseteil (hier Teil 4) am offenen Ende ein Außengewinde 5 und das andere Gehäuseteil (hier Teil 6) am offenen Ende ein komplementär zum Außengewinde ausgebildetes Innengewinde 7 aufweist.

In dem einen Gehäuseteil 4 ist ein Aufnahmeraum 14 für eine Batterie 16 mit einem federnden Kontakt 18 für den einen Pol 20 (hier für den Minuspol) der Batterie ausgebildet, wobei die Tiefe des Raumes 14 kleiner ist als die Länge der Batterie. Der Kontakt 18 ist über einen Draht 22 mit einem ersten ringförmigen Teil 24 einer ersten Elektrode 26 verbunden. Das Elektrodenteil 24 ist in einer am offenen Ende des Gehäuseteiles 4 mantelaußenseitig ausgebildeten umlaufenden Ausnehmung 28 angeordnet. Die Außenfläche des ringför migen Elektrodenteiles 24 fluchtet mit der Außenfläche des Gehäuseteiles 4.

In dem anderen Gehäuseteil 6 befindet sich beabstandet zu dessen freiem offenen Ende ein fester Kontakt 30 für den anderen Pol 32 (hier für den Pluspol) der Batterie. Hinter dem Kontakt ist eine von der Batterie gespeiste Schaltungsanordnung 34 zur Erzeugung elektrischer Spannungsimpulse angeordnet. Die Speisung dieser Schaltungsanordnung 34 erfolgt über einen im Bereich des geschlossenen Endes 12 angeordneten Reed-Kontakt 36, der in Ruhestellung geschlossen ist, also in dieser Stellung eine elektrische Verbindung über eine Verbindungsleitung 37 zwischen Batterie 16 und Schaltungsanordnung 34 herstellt.

Am offenen Ende des Gehäuseteiles 6 ist mantelaußenseitig eine umlaufende Ausnehmung 38 ausgebildet, in der ein zweites ringförmiges Teil 40 der ersten Elektrode 26 angeordnet ist. Die Außenfläche dieses Teiles 40 fluchtet mit der Außenfläche des Gehäuseteiles 6.

Die beiden, die erste Elektrode 26 (hier die Massenelektrode) bildenden ringförmigen Elektrodenteile 24, 40 werden beim Zusammenschrauben beider Gehäuseteile 4 und 6 in Kontakt miteinander gebracht.

Eine zweite ringförmige Elektrode 42 ist in einer in der Mantelfläche des Gehäuseteiles 6 ausgebildeten umlaufenden Nut 44 beabstandet zur ersten Elektrode 26 angeordnet. Die Außenfläche dieser Elektrode 42 fluchtet ebenfalls mit der Außenfläche des Gehäuseteiles 6.

Die von der Schaltungsanordnung 34 abgegebenen Spannungsimpulse werden über die beiden Elektroden 42 und 40 bzw. 26 abgegeben.

Die als Masseelektrode ausgebildete Elektrode 26 ist also zweigeteilt ausgebildet, so daß der Massekontakt für die Schaltungsanordnung 34 erst durch Zusammenschrauben der beiden Gehäuseteile 4 und 6 zustande kommt.

Zur Unterbrechung der Stromversorgung der Schaltungsanordnung 34 dient ein Magnet 45, der innerhalb einer zur Aufbewahrung des Stimulators 2 vorgesehenen Schatulle 48 an einem Ende 46 derselben angeordnet ist. Beim Einlegen des Stimulators in die Schatulle 48 wird der Reed-Kontakt 36 durch den Magneten 45 geöffnet, wenn der Stimulator mit seinem den Reed-Kontakt enthaltenden Ende 12 in das magnetseitige Ende 46 der Schatulle 48 eingelegt wird. Bei um 180° verdrehter Position des Stimulators innerhalb der Schatulle, in der sich der Reed-Kontakt entfernt vom Magneten 45 befindet, schließt der Reed-Kontakt, so daß an den Elektroden 42 und 26 die Impulsspannung der Schaltungsanordnung 34 anliegt. Diese Impulsspannung wird durch zwei in der Schatulle angeordnete Kontakte 50, 52 in dieser Position des Stimulators abgegriffen und mit Hilfe einer geeigneten Anzeigeeinrichtung 54, die im einfachsten Falle eine Leuchtdiode sein kann, angezeigt. Hierdurch ist somit eine Funktionskontrolle des Stimulators 2 möglich. Mit 56 ist ein Deckel der Schatulle bezeichnet.

Die Elektrode 42, aber auch die Elektroden 26, und 40, können aufgedampft sein; in diesem Falle kann auf die Nut 44 und die Ausnehmungen 28 und 38 verzichtet werden, was die Herstellung vereinfacht.

**Patentansprüche**

1. Elektrischer Stimulator für die Behandlung der weiblichen Inkontinenz mit einem in die Vagina einführbaren Gehäuse, in dem eine über eine in das Gehäuse einsetzbare Batterie gespeiste Schaltungsanordnung zur Erzeugung elektrischer Spannungsimpulse angeordnet ist, welche über zwei ringförmige, gehäuseaußenseitig angeordnete Elektroden nach außen abgegeben werden, und mit einem Schalter zum Ein- und Ausschalten des Stimulators, **dadurch gekennzeichnet,**

   daß das Gehäuse (8) aus zwei zusammenschraubbaren Gehäuseteilen (4, 6) besteht, an deren offenem Ende jeweils gehäuseaußenseitig ein ringförmig umlaufend angeordnetes Elektrodenteil (24, 40) angeordnet ist, die beim Zusammenschrauben der Gehäuseteile (4, 6) miteinander in Kontakt treten und eine erste Elektrode (26) bilden,

   daß das eine Gehäuseteil (4) eine Aufnahme für die Batterie (16) bildet und einen Kontakt (18) für den einen Pol (20) der Batterie aufweist,

   daß im anderen einen Kontakt (30) für den anderen Pol (32) der Batterie aufweisenden Gehäuseteil (6) die Schaltungsanordnung (34) zur Erzeugung der elektrischen Spannungsimpulse und ein in der Verbindungsleitung (37) zwischen Batterie (16) und Schaltungsanordnung (34) angeordneter, in Ruhestellung geschlossener Reed-Kontakt (36) angeordnet ist, der am geschlossenen Ende (12) innerhalb dieses Gehäuseteiles (6) positioniert ist,

   daß das die Schaltungsanordnung (34) aufweisende Gehäuseteil (6) gehäuseaußenseitig eine zweite, umlaufende Elektrode (42) aufweist, wobei die Ausgangsanschlüsse der Schaltungsanordnung (34) mit dieser zweiten Elektrode (42) und mit dem zweiten Elektrodenteil (40) der ersten Elektrode (26) verbunden sind, und

   daß für den Stimulator (2) bei Nichtgebrauch eine Einrichtung (48) vorgesehen ist, in die der Stimulator einlegbar ist, die ferner einen Magneten (45) aufweist zum öffnen des Reed-Kontaktes (36) in einer ersten Position des Stimulators innerhalb der Einrichtung und die mit zwei Kontakten (50, 52) ausgestattet ist, die in einer zweiten Position des Stimulators, in der der Reed-Kontakt (36) außer Wirkverbindung mit dem Magneten (43) steht, in Kontakt mit den Elektroden (26, 42) des Stimulators (2) stehen und zur Funktionskontrolle an eine Anzeigeeinrichtung (54) für die durch die Schaltungsanordnung (34) erzeugten Spannungsimpulse angeschlossen sind.

2. Elektrischer Stimulator nach Anspruch 1, **dadurch gekennzeichnet,** daß die geteilte Elektrode (26) als Masseelektrode ausgebildet ist.

3. Elektrischer Stimulator nach Anspruch 1, **dadurch gekennzeichnet,** daß die Einrichtung (48) eine Schatulle ist und daß der Magnet (45) innerhalb der Schatulle an einem Ende (46) derselben angeordnet ist.

4. Elektrischer Stimulator nach Anspruch 1, **dadurch gekennzeichnet,** daß die Anzeigeeinrichtung (54) eine Leuchtdiode ist.

5. Elektrischer Stimulator nach einem der vorhergehenden Ansprüche, **dadurch gekennzeichnet,** daß wenigstens eine der Elektroden (42, 26) auf das Gehäuse aufgedampft ist.

Fig.1

Fig.2